# EUROPEAN PATENT APPLICATION

(11) **EP 4 212 093 A1**
(43) Date of publication of application: **19.07.2023**
(21) Application number: 22153468.8
(22) Date of filing: 26.01.2022
(51) Int. Cl.: A61B 5/083, C07C 229/14, C08J 3/12

(54) **MOLECULAR SENSORS, METHOD AND KIT FOR THE DETECTION AND QUANTIFICATION OF CARBON DIOXIDE**

(30) Priority: 17.01.2022 GR 20220100037
(71) Applicant: ETH Zurich, 8092 Zürich (CH)
(72) Inventor: Morandi, Bill, 8707 Uetikon am See (CH); Green, Ori, 8046 Zürich (CH); Finkelstein, Patrick, 8049 Zürich (CH); Rivero Crespo, Miguel Ángel, 8057 Zürich (CH); Lutz, Marius Daniel Rinaldo, 8057 Zürich (CH); Bogdos, Michael Konstantinos, 8102 Oberengstringen (CH)
(74) Representative: P&TS SA (AG, Ltd.)

(57) **Abstract**

The present invention relates to a sensing method to specifically detect, measure and/or monitor carbon dioxide. It further relates to a probe adapted to specifically sense the carbon dioxide. The probe is of formula (I')

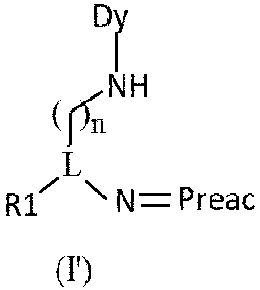

Wherein Dy is dye moiety, Preact is a reactive part to carbon dioxide and L is a spacer. The present invention also relates to a kit specifically adapted to sense, detect and/or measure the carbon dioxide.

## Description

### Technical domain

The present invention concerns a means of determining the presence of carbon dioxide (CO₂) in a test sample. It further concerns a reagent allowing to specifically detect the presence of carbon dioxide, as well as a testing kit specifically adapted to detect, measure and monitor the presence of carbon dioxide in a sample.

### Related art

Carbon dioxide is currently detected by means of very diverse methods including non-dispersing infrared (NDIR) sensors, positron emission tomography (PET) imaging, Severinghaus electrode, paper-based sensors, hydrogels, polymers, and MOFs. Most of these methods, are not well adapted for the determination of carbon dioxide at the scale of a laboratory experiment with high selectivity and sensitivity, preventing fast, accurate and reliable analysis of biological or chemical samples and reactions.

Activity-based sensing (ABS) has been used for sensing low weight reactive molecules, thus allowing to determine their contribution to some biological and chemical reactions. According to this principle, the analysed low weight molecule is involved in a chemical reaction so that it can be identified by a characteristic spectral measurement. Reactive nitrogen species and reactive oxygen species are well identified and monitored using ABS. Reactive carbonyl species such as carbon monoxide, phosgene and formaldehyde are also detected by this means. However, no accurate sensor has been developed so far, which is suitably specific and sensitive to carbon dioxide.

There is thus room to develop new and improved sensors which are specifically adapted to the identification and the measurement of carbon dioxide.

### Short disclosure of the invention

An aim of the present invention is thus the provision of a method adapted to specifically sense, monitor, and/or detect the presence of carbon dioxide in a sample. In particular, the aim of the present method is to selectively detect, sense or measure carbon dioxide in the presence of other reactive carbon species (RCS) such as carbon monoxide, carbon disulfide, and formaldehyde. The object is to reach a selectivity of carbon dioxide detection over the other reactive carbon species of more than 10, preferably more than 100 and more preferably more than 1000. The object is furthermore to reach a sensitivity of the carbon dioxide detection of a few ppm, such as 10 ppm.

Another aim of the present invention is to provide a functional molecule, which is highly reactive as well as highly specific to the carbon dioxide, while allowing its easy detection by a direct means such as a visual or spectral parameter.

Another aim of the present invention is to provide a tool, and/or a kit, or a testing arrangement, specifically adapted to determine the presence of carbon dioxide in a sample, in particular during a biological or chemical process or reaction. It is furthermore aimed at accurately monitoring the production of carbon dioxide and determining its variation of concentration over time in such a sample. The object is to provide such a kit or testing arrangement so that a limited number of analytical tools and analytical steps are necessary.

Another object of the present method is to provide a ready-to-use diagnostic method allowing to identify and/or characterize the amount of carbon dioxide in a biological sample, or its apparition rate.

According to the invention, these aims are attained by the object of the independent claims, which is further detailed in the dependent claims.

With respect to what is known in the art, the present invention provides an accurate means and product specifically adapted for sensing, measuring and monitoring the presence of carbon dioxide.

### Short description of the drawings

Exemplary embodiments of the invention are disclosed in the description and illustrated by the following drawings:
- Figure 1: Diagram of absorbance and fluorescence of a compound according to the present invention with and without carbon dioxide
- Figure 2: Diagram representing the correlation between the signal intensity and the carbon dioxide concentration for a compound according to the present invention,
- Figure 3: Diagram of comparative reactivity of a compound according to the present invention toward carbon dioxide and other reactive carbon species,
- Figures 4a, 4b, 4c: Diagram showing the effect of the reactive moiety of compounds according to the present invention,
- Figure 5a, 5b: Diagram showing the effect of the Dye moiety of compounds according to the present invention,
- Figure 6: example of inhibitory effect detection in an enzymatic reaction, using a compound according to the present invention.

### Examples of embodiments of the present invention

The present disclosure provides a method or a process specifically adapted for the detection of carbon dioxide in a sample. A sample is here understood as any part or subpart of material under test. A sample can be under different physical states such as gas, liquid, gel, suspension, emulsion or related states. It can be for example an air or a gas sample, or a liquid solution or a liquid suspension. A sample can be an aliquot or a portion of an environment such as seawater or air or a portion of a reactional mixture, in which a fixed amount of carbon dioxide is sensed and determined. In addition, a sample can denote any evolutive environment such as a reactional mixture in which the variation of carbon dioxide is sensed and monitored over time, in particular as a continuous detection. A sample can also include a production means wherein creation of carbon dioxide needs to be detected and/or monitored, either continuously or at predetermined periods of time.

The present sensing process includes a chemical reaction of carbon dioxide with a reagent used as a sensing molecule. To this end, it is considered as an activity-based sensing (ABS) method or process. In particular, the reaction rate between carbon dioxide and such a reagent should be as high as possible so that a very small amount of carbon dioxide and/or small amount of the reagent can be used. It is furthermore preferable that the reaction takes place at ambient temperature so that no heating step or activating step is necessary. The present reaction thus preferably occurs at a temperature as low as 20°C or 10°C or 5°C or lower. In a preferred embodiment, the reaction efficiently occurs through a large range of temperatures such as between -10°C to 80°C or between around 0°C and around 50°C. According to another preferred embodiment, the sensitivity toward carbon dioxide does not significantly vary over the above-mentioned temperature range. In other words, the sensitivity does not vary more than 10% or more than 5%. In addition or alternatively, the specificity or selectivity toward other reactive carbon species does not significantly vary through the temperature range above-mentioned. In particular, it does not vary more than 10% or 5%.

According to an embodiment, the present sensing process or method involves only one reagent used as a sensing molecule, meaning that the reagent reacts itself spontaneously in-situ with the carbon dioxide so as to provide a direct readable result. Preferably, no co-reactant or catalyst is necessary. Thus, the present sensing method does not necessitate adding other chemicals.

The reagent, preferably used as a single reagent, may be stored and supplied as such, so that it can be directly used for a carbon dioxide measurement, detection or monitoring operation. The reagent is thus stable enough at room temperature or at a manageable temperature comprised between 5°C and 30°C, or between 10°C and 30°C. Under such condition the present method is free of any preparation step dedicated to provide a functional reagent.

Alternatively, the reagent may be supplied as two or more distinct separate subparts. This may be necessary for stability reasons, or for safety reasons, or for any other reasons such as regulation constraints. In particular, each separate subparts of the reagent may correspond to a dye moiety or a dye moiety precursor, a reactive moiety or a reactive moiety precursor, or a linker or a linked precursor adapted to adsorb the reagent on a surface, or any other adequate subpart of the reagent. Preferably, all these subparts can be combined together so that the functional reagent is produced. Preferably, the combination of the subparts is easily handled and can be made by merely mixing the subparts in a common solvent, or solubilising one of the subparts into the other. Under these conditions, the sensing method may comprise a preliminary step of preparing a functional reagent based on predetermined subparts of a reagent. According to a specific embodiment, several subparts are supplied as a kit, wherein different dye moieties can be chosen to react with different reactive moieties so that a diversity of reagent can be produced at the point of carbon dioxide measurement. For example, depending on the specific needs a user may select one dye moiety or one dye precursor over a choice of several dye moieties or dye precursors and combine it with a predetermined reactive moiety or reactive precursor. Furthermore, the reactive moiety or reactive precursor may be selected over a choice of several possible reactive moieties or reactive precursors. Thus, the method may comprise a step of selecting one or both of the dye and the reactive moiety of the reagent, before combining them and produce the corresponding functional reagent. In case other subparts are available, they may be equally subject to a preliminary selection over several alternatives. As such a subpart related to a spacer bounding two different subparts of the reagent can be selected over several alternatives. A linker adapted to bound the reagent on a surface can be selected over several alternatives, depending for example on the nature of the support. Plenty of variations are thus available, although not specifically described here. It is thus understood that the present sensing method or process, allows to adapt or adjust the reagent at the point of measurement or detection.

The step of combining the subparts, if applicable, may include adding one or more additives allowing an easy reaction between the subparts. Such additives may relate to a catalyst or a buffer or any other suitable adjuvant. Preferably, the preparation of the reagent based on its subparts is made at ambient temperature, such as comprised between 5°C and 30°C or between 10°C and 25°C, with a mere manual agitation in a vial. It preferably occurs instantaneously or in a time frame of a few minutes such as less than 5 minutes or 15 minutes.

The preparation of the reagent may be performed previously to the carbon dioxide measurement or detection operation. Advantageously, it can be done in-situ. In that case, the sensing method or process comprises the step of adding the subparts of the reagents to the sample or in contact with the sample, instead of the reagent itself, so that the reagent is produced while the carbon dioxide detection or measurement is initiated.

The reagent used as a sensing molecule is selective or specific for carbon dioxide. This means that the reaction rate has a clear statistically significant difference compared with other reactive carbon species such as carbon monoxide, carbon disulfide, and formaldehyde. The reaction rate may be for example at least 10, or 100 or 1000 or 10000 times higher with carbon dioxide compared with other reactive carbon species. In a preferred embodiment, it is specific from the carbon dioxide, so that it does not produce the desired fluorescence with other reactive carbon species. The present sensing method or process is thus applicable for a sample comprising several reactive carbon species, and in particular one or more of carbon monoxide, formaldehyde and carbon disulfide, in addition to the sensed carbon dioxide. Furthermore, the present sensing method is also applicable to reaction mixtures involving other reactive species than carbon dioxide, without disturbing the reaction itself. Therefore, the present method allows to follow a reaction, such as chemical reaction, enzymatic reaction or biological reaction without disturbing it.

The reagent of the present invention comprises a reactive moiety adapted to spontaneously react with carbon dioxide. According to a preferred embodiment, the reactive moiety is designed to irreversibly react with carbon dioxide so as to definitely trap it under the reaction conditions. The reactional conditions denote here the conditions under which the carbon dioxide detection or measurement is performed. This provides the advantage to limit or avoid any exchange or equilibrium with other reactive carbon species potentially present in the sample. The present sensing method or process comprises a step of contacting one of such a reagent or the subparts thereof, with the sample so that the reagent can react with the carbon dioxide present in the sample.

According to a preferred embodiment, the reactive moiety of the reagent is a phosphorous-based reactive moiety Preac. Preferably, the reactive moiety is a phosphine such as a triarylphosphine, like triphenylphosphine, an alkyldiarylphosphine or an alkyldiphenylphosphine like a methyldiphenylphosphine or an ethyldiphenylphosphine, or a dialkylarylphosphine like dimethylphenyl phosphine, and related chemical reagents. The alkyl here denotes any linear or branched alkyl comprising from one to 10 carbon atoms. They thus include groups such as methyl, ethyl, propyl, isopropyl, butyl, isobutyl, tertbutyl, cyclohexyl, adamantyl. The phosphorous-based reactive moiety Preac equally denotes phosphines which are free of aromatic groups, and bearing only alkyl groups such as those above-described. In the phosphorous-based reactive moiety Preac each one of the mentioned groups, either alkyl or aromatic may be independently substituted by one or several groups such as Halogen, NO₂, CF₃, NH₂, SO₂, COH, COOH, or related groups.

More preferably the phosphorous based reactive moiety Preac is bound to an amine of the reagent so as to form an iminophosphorane. As such the phosphorous-based reactive moiety Preac can easily be replaced by a group CO upon reaction of the carbon dioxide. The reactivity of the phosphorous-based reactive moiety Preac toward the carbon dioxide, including its reaction rate and its selectivity with regard to the other reactive carbon species, can be tuned or adjusted with the substituents of the phosphorous-based reactive moiety Preac. For example, one or several of the phenyl groups of the above-mentioned phosphines can independently comprise 1, 2, 3 or 4 substituents such as nitro, halogen, CF₃, carbonyl, sulfoxides or related chemical groups.

Once the carbon dioxide has reacted upon the reagent, leading to the replacement of the phosphorous-based reactive moiety Preac by a group CO, an intramolecular reaction occurs. The intramolecular reaction is designed to provide a visible change in the reagent molecule, as it will be better described below. Preferably, the intramolecular reaction is performed under the reactional conditions between the CO group originating from the surrounding carbon dioxide and a reactive group or a heteroatom present in the reagent molecule. Such heteroatom or reactive group can be selected among an amine, such as a secondary amine, an alcohol, a thiol, or any suitable reactive chemical group. The present sensing process or method thus comprises a step of modifying in-situ the reagent upon reaction with the carbon dioxide, so as to reveal its presence in the sample. The intramolecular reaction may denote any suitable intramolecular reaction adapted to structurally modify the core structure of the reagent. It can denote for example a cyclisation or a rearrangement, preferably a cyclisation. Although an intermolecular reaction is not fully excluded, it is currently considered not preferably.

The reagent used in the present sensing process or method, further comprises a dye moiety. The dye moiety Dy is here understood as a portion of the reagent exhibiting optical and/or spectral activities. Any light related activity may be considered regarding the properties of the dye moiety Dy. Such optical and/or spectral properties may relate for example to light absorption, light emission, phosphorescence and fluorescence, in specific spectral ranges, or at specific wavelengths, or a colour change in the visible, IR or UV range. Other light related characteristics such as polarized light deviation or Raman effect may also be considered. Although optical and/or spectral parameters are preferred, the present method may equally be based on other measurable parameters such as magnetic-related parameters. It is here understood that the properties of the dye moiety Dy are preferably directly measurable from the sample. This thus excludes any separate analytical step involving chemical separation, chemical purification, and related analytical additional steps.

The properties of the dye Dy in the reagent, are designed to change once the reagent has reacted with the surrounding carbon dioxide. More particularly, the properties of the dye Dy moiety change once the intramolecular reaction above-mentioned occurs, leading to a change in the core molecular structure, impacting the properties of the dye moiety. According to a preferred embodiment, the dye moiety Dy is directly bound to the reactive group or the heteroatom involved in the intramolecular reaction. The term *"directly bounded"* means here that the optical and/or spectral properties of the dye moiety Dy remain localised as close as possible to the reactive group or heteroatom involved in the intramolecular reaction so as to be modified by the structural change resulting from the intramolecular reaction. This excludes for example any spacer between the dye moiety Dy and the reactive group or heteroatom involved in the intramolecular reaction.

In a preferred embodiment, the reactive group or heteroatom involved in the intramolecular reaction is an amine and the dye moiety Dy comprises at least one double bond in position α to this amine to which it is bounded. Under such configuration, the double bond is conjugated with the free electron lone pair of the nitrogen atom of the amine. The dye moiety Dy preferably comprises other unsaturation such as delocalised double or triple bonds, responsible for optical and/or spectral properties. In particular, the dye moiety Dy preferable comprises one or several aromatic cycles such as phenyl, naphthyl, pyridine, quinoline, quinoxaline, phenanthrene and related aromatic elements.

Therefore, the present sensing process or method comprises the step of contacting a reagent as above described, wherein the dye moiety Dy has first optical and/or spectral properties, to a sample comprising carbon dioxide, so that the reagent specifically or selectively reacts with the carbon dioxide and is further involved in an intramolecular reaction leading to a modified reagent, wherein the dye moiety Dy has second optical and/or spectral properties. The first and the second optical and/or spectral properties are different from each other so that the modified reagent can easily and immediately be sensed.

According to a preferred embodiment, the optical and/or spectral properties relate to fluorescence. The first optical and/or spectral properties can consist of no fluorescence or a fluorescence at a first wavelength. The second optical and/or spectral properties can consist of the presence of fluorescence, or a more intense fluorescence or a fluorescence at a second wavelength different from the first wavelength. Preferably, the dye moiety Dy in the reagent presents no fluorescence at a predetermined wavelength, such as 455 nm or another specific wavelength, and presents a measurable fluorescence at this specific wavelength once the reagent has reacted with carbon dioxide. According to such an arrangement, the presence of carbon dioxide in the sample switches ON the fluorescence of the reagent and can thus be detected. One or several wavelengths can be specifically considered to determine a "switch ON" once fluorescence appears at such specific wavelengths.

The reversed arrangement can also be envisaged. For example, the first optical and/or spectral properties can consist of a fluorescence at a predetermined wavelength, which is switched OFF upon reaction with the carbon dioxide. In this configuration, fluorescence disappears when all the reagent has reacted. This allows for example to detect that a predetermined amount of carbon dioxide has been consumed, based on a predetermined amount of reagent.

The sensing process or method thus comprises the step of detecting one or both of the first and the second optical properties of the dye moiety Dy. To this end, an adequate spectral or optical measurement device can be used. In case only the presence of carbon dioxide is intended to be detected in a sample, no specific optical device is necessary when the carbon dioxide switches ON the reagent. A mere visual control is enough to determine the presence of carbon dioxide. Also, a colour shift may occur, which is visually identified. Such a method is well adapted to easily detect the occurrence of carbon dioxide, either for safety reasons or for a chemical process follow up. In case the first and second optical and/or spectral parameters consists of two different wavelengths or two different spectral profiles, then a detecting device may be necessary.

According to another embodiment, the first and/or second optical and/or spectral properties are correlated to the amount of carbon dioxide in the sample. For example, the intensity of the corresponding optical or spectral signal is proportional or related to the concentration of carbon dioxide. The present sensing process or method can thus include a step of measuring the amount of carbon dioxide or continuously monitoring its generation in a sample. Continuously monitoring means that a continuous measurement is performed, without interruption. Otherwise, the monitoring is performed step by step, by sensing and measuring the carbon dioxide at predetermined periods of time, and comparing the obtained values over time.

The step of detecting one or both of the first and the second optical properties of the dye moiety Dy thus denotes either merely detecting the presence of carbon dioxide in a sample, or detecting its presence and measuring its concentration over time. Carbon dioxide is detected when it reaches a certain amount or a certain concentration. The present method allows to detect carbon dioxide at very low proportions, as low as few ppm, such as below 10 or 5, or 1 ppm.

The detection step can be performed with any suitable detection device such as confocal microscopes and plate readers.

The sensing method or process according to the present invention may use more than one reagent here described. All the above-described steps can equally be applied with one reagent or with two different reagents, concomitantly used. For example a first reagent may be selected because of its improved sensitivity toward carbon dioxide and thus allow the detection of low concentrations of carbon dioxide. In case the monitoring should be performed over a large range of carbon dioxide concentrations, a second reagent more accurate for greater carbon dioxide concentrations can be used in combination. Similarly, detecting or measuring carbon dioxide over an extended range of pressures and/or temperatures may need the use of different reagents showing specific responses at specific subranges of temperatures or pressures. A more accurate detection and/or measurement of carbon dioxide can thus be obtained. In that case, two different reagents can be selected based on a variety of parameters such as their stability, sensitivity, selectivity, solubility, etc...

According to another aspect, the above-described method or process can be successively iterated with different reagents.

Depending on the nature of the experiment, the sensing process may comprise one or several additional steps such as the following:
- dissolving the reagent in a solvent so as to prepare a solution having a predetermined concentration of reagent. The reagent can be supplied as a powder or as a liquid. As such, it may be not usable to detect or monitor the carbon dioxide. Solubilising it in a suitable solvent allows to prepare a calibrated solution the optical and/or spectral properties of which can be directly sensed and monitored. When the concentration of reagent is known, the amount of sensed carbon dioxide can be determined. The solvent can be a dedicated solvent, such as water, distilled water, dimethyl sulfoxide, ethanol, or any other dedicated solvent and mixture thereof.
- adsorbing the reagent on a solid surface such as a glass or a paper surface. To this end, a solution of reagent can be sprayed on the solid support, or the surface can be immersed in a solution of reagent. Other means such as capillarity can be used to make the reagent reacting with the sample from a solid support.

According to a preferred embodiment, the present process for the detection and/or monitoring of carbon dioxide in a sample comprises the step of contacting said sample with a reagent of formula (I') 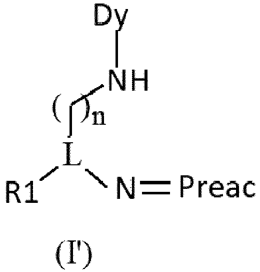 so that a compound of Formula (II') is obtained when reacting with the surrounding carbon dioxide: Wherein : 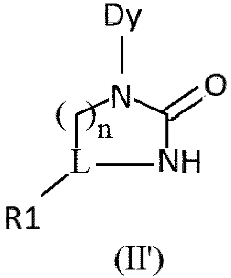
- Dy denotes a dye moiety having first optical and/or spectral properties in the compound of Formula (I) and second optical and/or spectral properties in the compound of Formula (II),
- Preac denotes a phosphorous based reactive moiety, on which CO₂ reacts in an irreversible manner,
- L denotes an internal spacer selected from a linear or branched carbon chain comprising from 1 to 10, preferably from 1 to 6 carbon atoms being either saturated or mono- di- or tri-unsaturated, or a cyclic chain comprising from 4 to 7, or from 5 to 6 carbon atoms, being either saturated or mono- or di- unsaturated, or an aromatic group such as a phenyl, or a heteroaromatic group such as pyridine, (benzo)furan, (benzo)thiophene, or pyrrole, wherein, where applicable, in the flexible linear or branched carbon chain and in the non-aromatic cyclic chain, from 1 to 3 carbon atoms, can independently from each other optionally be replaced by a heteroatom selected from -O-, -S-, -NH-,-CO-, -NHCO- or -COO- or substituted by a group -OH, -NH₂, halogen, NO₂, or alkyl comprising from 1 to 3 carbon atoms.
- R1 denotes from 1 to 4 groups independently selected from, H, C₁-C₆ alkyl, C₁-C₆ alkenyl, C₁-C₆ alkynyl, or denotes 2 groups forming a fused saturated, none-saturated or aromatic C₄-C₈ ring with the spacer L to which they are linked, wherein from 1 to 3 carbon atom, where applicable, are independently from each other optionally substituted by a group selected from -COO-, -CO-, NH-, -O-, -S- or substituted with a group selected from -OH, SH, NH₂, NO₂, halogens, or denotes a macromolecule such as a DNA, RNA, peptide, protein, enzyme, and
- n denotes an integer selected from 0, 1, 2 or 3.

According to a more preferred embodiment, the present process for the detection and/or monitoring of carbon dioxide in a sample comprises the step of contacting said sample with a reagent of formula (I') wherein the spacer L is a phenyl group. It can be represented by a compound of Formula (I) 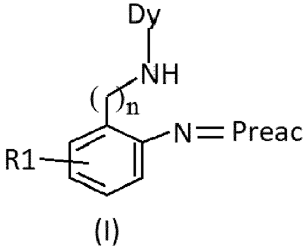 so that a compound of Formula (II) is obtained when reacting with the surrounding carbon dioxide: 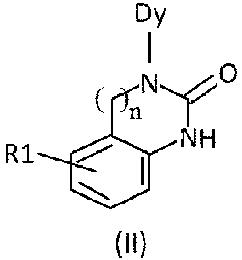 Wherein Dy, Preac, R1 and n are as above defined.

Preferably, in formulae (I) and (II), R1 denotes from 1 to 4 groups independently selected from, H, C₁-C₆ alkyl, C₁-C₆ alkenyl, C₁-C₆ alkynyl, or denotes 2 groups forming a fused saturated, non-saturated or aromatic C₄-C₈ ring with the phenyl group to which they are linked.

Preferably, in formulae (I) and (II), n denotes 0, 1 or 2, more preferably 0 or 1.

According to a specific embodiment, R1, in any of compounds of Formulae (I) and (I') can denote or comprise a linker adapted to bound the reagent on a solid surface such as a glass, a metal or cellulosic surface. Such a linker can comprise at its free end a group reactive to -SiOH, -SH, -OH, or related groups present on the solid surface. To this end, the linker can bear at its free end a group such as carbonyl, aldehyde, amine, or any suitable reactive group so that a covalent bond can be established.

Alternatively, one of the **R1** groups in the compounds of Formulae (I) and (I') can denote a macromolecule such as a DNA, RNA, peptide, protein, enzyme or any biochemical macromolecule.

Alternatively, one or several of the **R1** group can be adapted to improve the solubility of the reagent in some dedicated solvents. Thus, the groups C₁-C₆ alkyl, C₁-C₆ alkenyl, C₁-C₆ alkynyl, and fused saturated, unsaturated or aromatic C₄-C₈ ring above-mentioned may be substituted with polar groups such as -OH, SH, NH₂, NO₂, halogens and related groups so that solubility in water or in a polar solvent is improved.

The dye moiety Dy comprises at least one double bond in position α to the nitrogen atom to which it is bounded, so that said double bond is conjugated with the free lone pair of the nitrogen atom in the compounds of Formulae (I), (I').

The dye moiety Dy is preferably selected from one of the following moieties **Dy1, Dy2 and Dy3:**

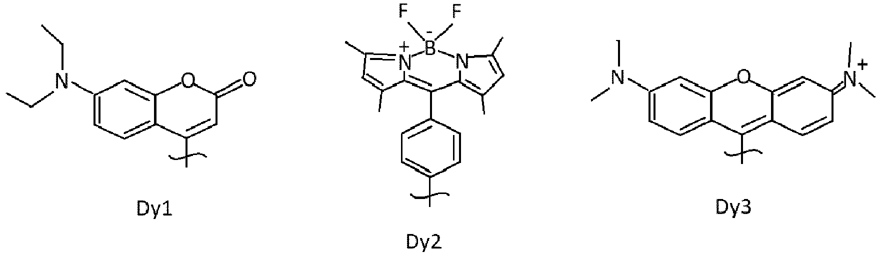

The group **Preac** denotes any phosphorous-based reactive moiety above mentioned, in particular a triarylphosphine, an alkyldiaryl phosphine, a dialkylarylphosphine or a trialkylphosphine, wherein alkyl and aryl are as defined herein. According to a preferred embodiment, the group **Preac,** relative to the reactive moiety, denotes a phosphine selected from PMePh₂, PEtPh₂, PMe₂Ph and PPh₃ wherein "Ph" independently denotes an unsubstituted phenyl group or a phenyl group substituted with 1 to 4 substituents selected from a linear or branched C₁-C₆ alkyl, C₂-C₆ alkenyl, C₂-C₆ alkynyl, NO₂, halogen and CF₃.

In the reagent, the reactive moiety **Preac** and the reactive group or heteroatom involved in the intramolecular reaction are linked through the spacer L adapted to allow the intramolecular reaction. Such a spacer may be a flexible linear or branched carbon chain comprising from 1 to 10 carbon atoms, or from 2 to 6 carbon atoms It is preferably a cyclic chain comprising from 4 to 6 carbon atoms, and more preferably an aromatic group such as a phenyl or a pyridine group, as disclosed in Formulae (I), (II) and (III). Where applicable, in the flexible linear or branched carbon chain and in the non-aromatic cyclic chain, from 1 to 3 carbon atoms, can independently from each other optionally be replaced by a heteroatom selected from -O-, -S-, -NH-,-CO-, -NHCO- or -COO- or substituted by a group -OH, -NH₂, halogen, NO₂, or alkyl comprising from 1 to 3 carbon atoms.

When reacting with the surrounding carbon dioxide, the compound of formula (I) provides an intermediate compound of Formula (III) below, which immediately provides an intramolecular reaction leading to a compound of Formula (II). 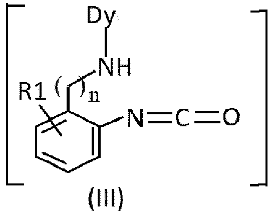 wherein Dy, R1 and n are as above defined. The intramolecular reaction contributes to the optical and/or spectral change of the dye moiety Dy. Preferably, both reaction with carbon dioxide and intramolecular reaction are irreversible.

Of course, the reaction is similarly applicable with a linker L different from a phenyl group, as long as the intramolecular reaction is possible. As such a compound of Formula (I') produces an intermediate of Formula (III') 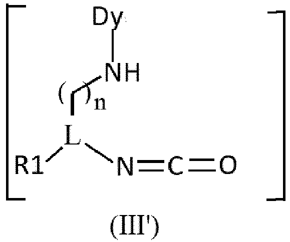 Wherein L, Dy, R1 and n are as above defined.

The present invention also comprises a reagent as described above. The present reagent is dedicated to specifically react with carbon dioxide and to provide an in-situ visible structural change upon its reaction with carbon dioxide. To this end, it comprises a reactive moiety and a dye moiety the property of which changes upon reaction with carbon dioxide. It further comprises a reactive group or a heteroatom adapted to provide an intramolecular reaction once the reactive moiety has reacted with carbon dioxide. The reactive group or heteroatom is bound to the reactive moiety through a suitable spacer **L.** The reactive moiety is preferably the phosphorous based reactive moiety **Preact** defined above. The dye moiety is preferably one of the dye moieties **Dy** above defined.

As mentioned above, any combination of the dye moieties **Dy** and the reactive moieties **Preac** can be envisaged so as to tune the reagent according to the needs.

In a preferred embodiment, the reagent of the present invention are selected among the following (I1), (I2), (I3), (I4), (I5) and (I6):

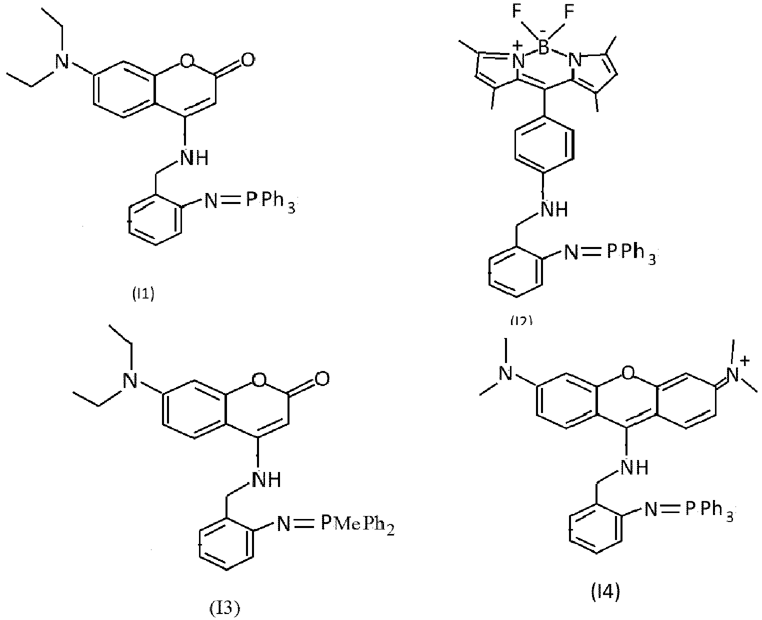

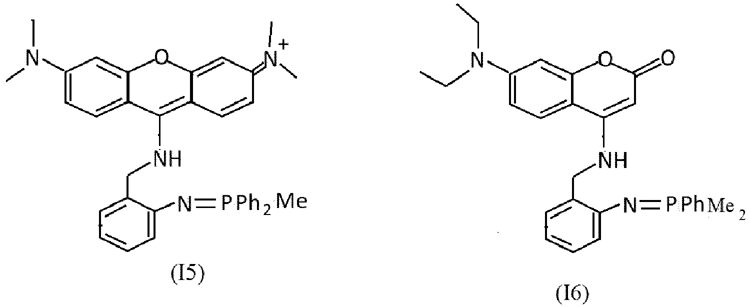

The present reagent can be prepared by reacting a precursor of the dye moiety with the assembly comprising a precursor of the reactive moiety and the reactive group or heteroatom, bounded together through a suitable spacer **L.**

For example, for reagents related to compounds of formula (I), a compound of Formula (la) : 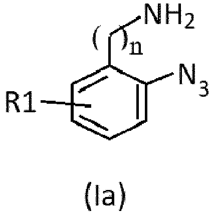 can be reacted with a compound of Formula (lb) : 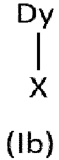 to provide a compound of Formula (Ic):

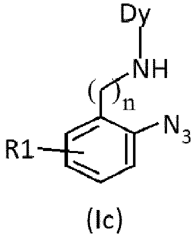

Wherein Dy, R1 and n are as defined above and wherein X denotes a suitable electrophile such as a halogen, preferably a Cl or Br atom, a carbonyl, a thiocarbonyl, a triflate mesylate or a group O-SO₂-R, wherein R denotes any suitable substituent such as a linear, branched or cyclic alkyl comprising from 1 to 10 carbon atoms and optionally substituted or an aryl group.

Such a reaction may be performed in a suitable solvent such as ethanol, DMSO, DMF, ethyl acetate, water. Suitable additives such as bases can be used. For example, the above reaction can be conducted in the presence of triethylamine in ethanol at a temperature comprised between 60°C and 120°C during 10 minutes to 20 hours.

The obtained compound comprises a precursor of the reactive moiety, which needs to be transformed into the suitable reactive moiety by one or several steps. For reagents related to compounds of Formula (I), the process can comprise the step of reacting a compound of Formula (Ic) with a phosphorous based moiety Preac, as above-defined, in DMSO at a temperature comprised between 20°C and 80°C so that corresponding compound of Formula (I) above mentioned is obtained.

The present invention further relates to a kit specifically adapted for the detection, the measurement and/or the monitoring of carbon dioxide. The kit according to the present invention comprises at least one reagent, as described above. Preferably, the kit comprises more than one reagent, each one being adapted to different testing conditions, or having different responses. For example, a reagent may be considered not suitable if its optical or spectral properties overlap with those of the sample itself so that detection of the optical or spectral changes is not easy. The kit can comprise at least some indications concerning the optical and/or spectral properties of the reagent or the reagents. It can in addition comprise information related to a correlation between the optical or spectral signals and the carbon dioxide concentrations. The kit can comprise for one or several reagents the subparts of the corresponding reagents, separately supplied so as to allow their combination.

The reagents, or subparts thereof, can be supplied under any suitable form, such as a liquid in a vial, or a solid or a powder or a gel in a suitable recipient. The reagent or subparts thereof can be supplied as a predetermined amount, corresponding to a specific usage. For example, it can be supplied as compressed solid having a predetermined weight and easily dispersible in a sample. Alternatively or in addition, the kit can comprise one or several solvents, or one or several vials adapted to receive a solvent, each one having a predetermined size so that a reagent can be prepared at a predetermined concentration. Alternatively, the reagents or some of the reagents of the kit can be already adsorbed on a solid surface such as a glass or paper-based surface. As such, contacting the solid surface with a sample can provide a visual change of the surface such as a colour apparition, indicating the presence of carbon dioxide.

The present kit can comprise in addition a light emission device such as a UV light emission device adapted to irradiate the sample and reveal the responsive signal of the reagent.

### Example 1: spectral measurement

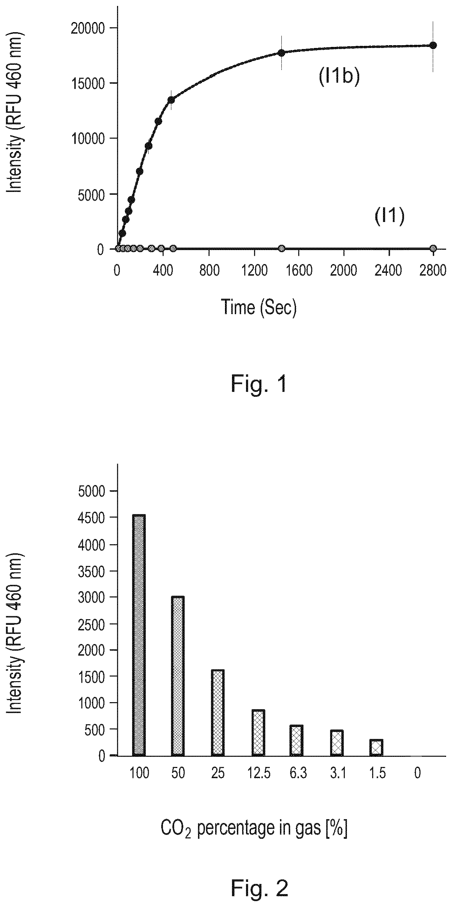

Compound (I1) was solubilised in DMSO and involved in carbon dioxide detection experiment, resulting in the compound of formula (I1b). 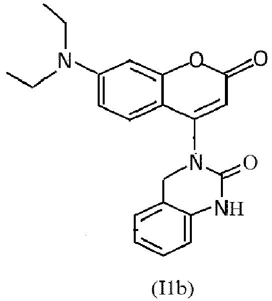 (I1) and (I1b) exhibit distinct absorbance and emission maxima, making them suitable CO₂ detection. Figure 1 shows the observed signal for both (I1) and (I1b) at 455 nm in the presence of an increase in concentration of carbon dioxide.

The compound (I1) was placed in the presence and absence of CO₂ (Figure 2). In the absence of carbon dioxide, a negligible fluorescent response was observed, while upon bubbling carbon dioxide a strong fluorescent response, which further increased over time, was detected with a maximal emission at 455 nm, identical to the spectra of (I1b). The turn-on response was saturated at a signal-to-noise ratio of 260. This high value demonstrates the high sensitivity of the fluorescent approach.

### Example 2: Kinetic experiment

The reaction kinetics were investigated with the same compounds of Formulae (I1) and (I1b) by measuring fluorescence at different time points using varying concentrations of either (I1) or carbon dioxide. The reaction shows a first-order dependence for both (I1) and carbon dioxide, pointing towards the initial intermolecular isocyanate formation as the rate-determining step.

Gas mixtures with different ratios of CO₂/N₂ were bubbled into solutions containing (I1) at a constant volume and rate (governed by a mass flow controller). (I1) showed a CO₂ dose-dependent response. It could sense a mixture of 1.5% CO₂ with a signal to noise of -20. Both polar and nonpolar solvents have been used, including water, boding well for a wide range of interdisciplinary applications, without diverging results.

The compound of Formula (I2) was used for sensing atmospheric CO₂ levels. (I2) was exposed to Air/N₂ mixtures. The limit of detection (Background + 3σ) was a 1.5% air (98.5% N₂) mixture. As air contains ∼0.04% of CO₂, this shows that (I2) is capable to detect carbon dioxide at concentrations of 0.0006% (∼6.4 ppm) of the gas mixture.

### Example 3: selectivity experiment

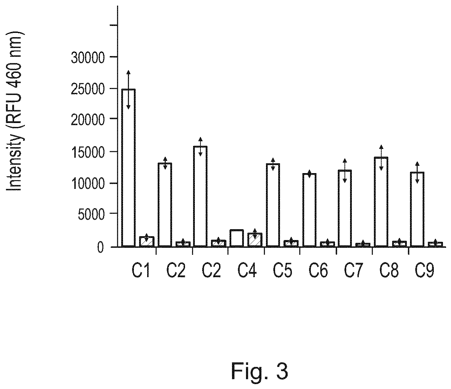

The selectivity of (I1) was compared to a panel of relevant reactive carbon species shown in Figure 3. Compound (I1) did not produce any significant fluorescent response to most of the tested reactive carbon species other than to CO₂. Selectivity for CO₂ over otherwise more reactive compounds, was therefore demonstrated.

The reactive, potentially competing small molecules mentioned in figure 3 were the following:

| | |
|---|---|
| C1 | SO₂ |
| C2 | CO |
| C3 | AcCl |
| C4 | COCl₂ |
| C5 | CH₂O |
| C6 | Para-formaldehyde |
| C7 | Acetone |
| C8 | CS₂ |
| C9 | None |

After 60 minutes, CO₂ was added to all samples and the emission was measured again. The successful activation of the sensor with CO₂ in the presence of the tested reactive carbon species shows that these compounds did not negatively interfere with the sensor, apart from tris-phosgene which gave a complex mixture of products.

### Example 4: effect of the reactive moiety Preac

The ability to tune the reactivity of the sensor towards carbon dioxide was investigated. Compounds of Formula (I1), above-mentioned was compared to compounds of Formulae (I6) and (I3) below:

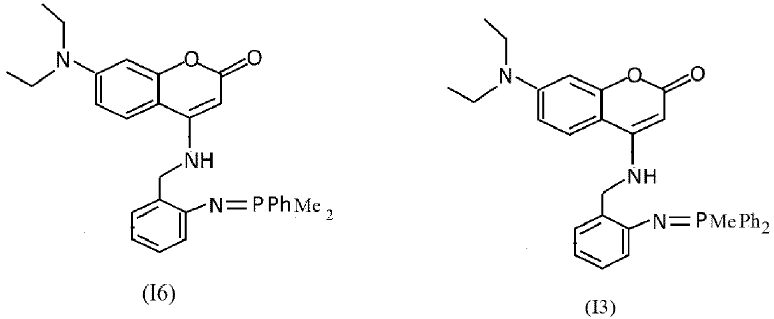

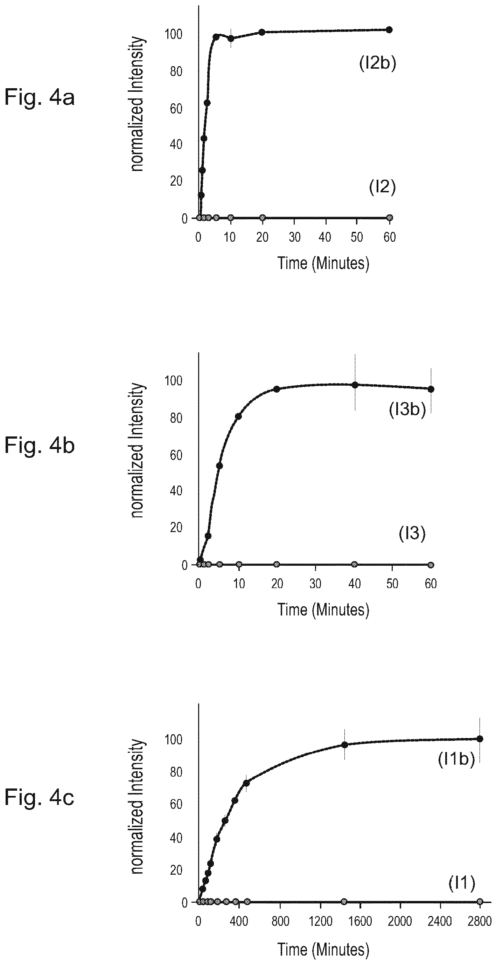

Figures 4a, 4b and 4c provide comparative data regarding their reactivity toward carbon dioxide. In these figures the corresponding compounds (I2b) and (I3b) correspond to the compounds (I1b) wherein the PPh₃ group is respectively replaced by the PPhMe₂ and PPh₂Me groups.

### Example 5: effect of the dye moiety Dy

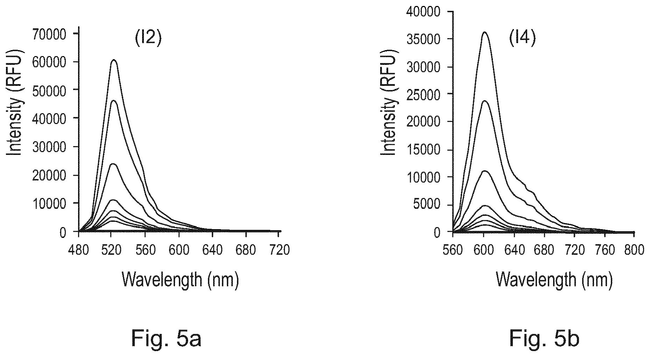

The two molecules of formulae (I2) and (I4) below were involved in a carbon dioxide detection experiment. The results are shown in figures 5a and 5b.

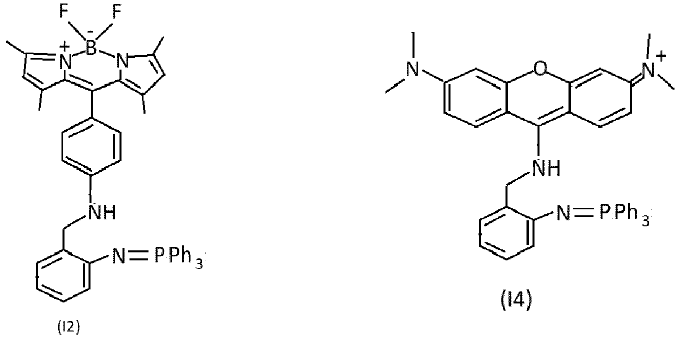

### Example 6: Carbon dioxide detection in a liquid sample

Compound of Formula (I1) was involved in a microscale experiment of catalytic decarboxylative reaction. Benzylic decarboxylative process was investigated using standard protocols wherein the solvent contains 100 µM of (I1). During the course of the reaction, the released carbon dioxide was captured by the sensor, giving rise to a fluorescent signal. The fluorescent measurement nearly instantaneously indicated the reaction. In the presence of catalytic amounts of a base, para-nitrophenyl acetic acid was converted to its corresponding decarboxylated product. In the absence of base, no product was obtained. In the reactions conducted in the presence of a base, the decarboxylation took place and exhibited a significant increase of the fluorescent signal compared to those without base.

In addition, the time-dependent experiment showed a correlation between the generated fluorescent signal and the reaction progress, as monitored by HPLC.

### Example 7: enzymatic monitoring

Fluorescence real-time monitoring of enzymatic activity was investigated for inhibitors and drugs screening. Urease, a nickel-based metalloenzyme that converts urea to ammonia and CO₂, was used in presence of the compound of Formula (12) above-mentioned, which is water-soluble and brightly fluorescent in water. The enzymatic assay was conducted in PBS at pH = 7.4, with altering concentrations of both the urea and urease (from *Canavalia ensiformis* (Jack bean). A 96 well plate was used for the setup and the fluorescent response was measured over time using a plate reader.

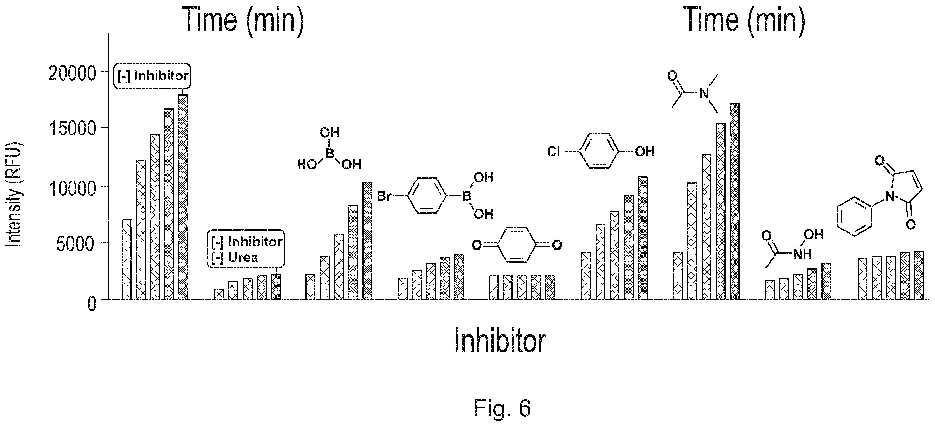

Figure 6 shows that the sensor was able to distinguish between all altering levels of both urea and urease, while in the absence of either urea or urease only a negligible signal was observed.

### Example 8: Cell imaging

The selectivity of the compound (14) was evaluated against common analytes in cells. HeLa cells were treated with compound (I4) [5µM] for 10 minutes, followed by washing thrice to remove excess sensor. Then, as an exogenous CO₂ model, the cells were incubated under ambient air or at 5% CO₂ and imaged at different time points. A significant time-dependent fluorescent increase in the red channel and decrease in the green channel was observed. Measurements of the fluorescent signals arising from within the cells were found to exactly match the spectral fingerprint of the activated sensor, confirming the reaction of CO₂ with the compound (I4). In addition, time-dependent response of cells that were incubated under ambient conditions was attributed to basal levels of CO₂ production by metabolic processes.

### Experimental details

All used reagents were purchased from common suppliers and used as received, unless noted otherwise. Solvents used for syntheses were of puriss grade and technical grade solvents were used for column chromatography.

Unless stated otherwise, all reactions were conducted in oven or flame-dried glassware equipped with a Teflon-coated magnetic stirring bar. Reactions were monitored by thin-layer chromatography (TLC) unless noted otherwise. TLC was performed on Merck silica gel 60 F254 TLC aluminium plates and visualized with UV fluorescence quenching and/or potassium permanganate (KMnO₄) stain.

Column chromatographic purification was performed as flash column chromatography (FCC) using silica gel (SiliCycle, 40 - 63 µm or Sigma Aldrich 40 - 60 µm) and an applied air pressure of 0.3 - 0.5 bar. The yields given refer to chromatographically purified compounds unless indicated otherwise.

NMR spectra were recorded on Bruker Avance III 300 MHz, Bruker Avance III HD 400 MHz, Bruker Neo 400 MHz or Bruker Neo 500 MHz spectrometers at room temperature. ¹H and ¹³C spectra were referenced to residual solvent peaks. The multiplicities are abbreviated as s (singlet), d (doublet), t (triplet), q (quartet), p (quintet), hept (heptet), m (multiplet) and combinations thereof.

High resolution mass spectrometric (HRMS) data was obtained using electrospray ionization (ESI) on a Bruker maXis - ESI-Qq-TOF-MS and are reported in m/z.

### Coumarin based probe:

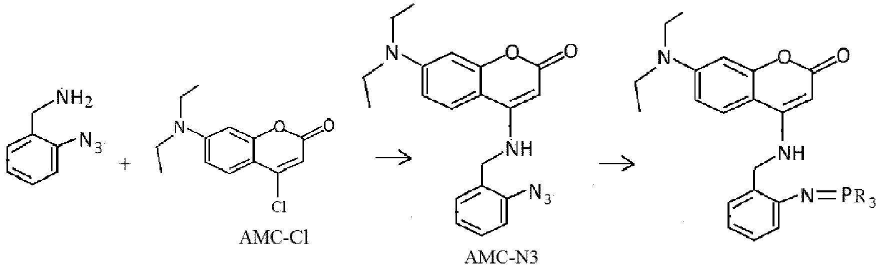

### AMC-N₃

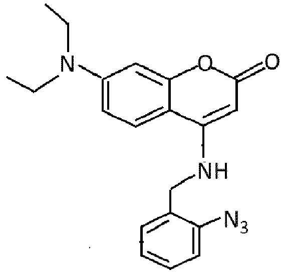

AMC-Cl (200.0 mg, 795 µmol, 1.0 equiv) and (2-azidophenyl)methanamine2,3 (147 mg, 993 µmol, 1.25 equiv) were suspended in EtOH (2ml) in an 8ml vial and NEt₃ (0.554 ml, 3.97 mmol, 5.0 equiv) was added. The mixture was heated to 100 °C for 3h and monitored by TLC (EtOAc:hex 30:70). Upon full conversion of the starting material, the resulting precipitate was filtered, washed with EtOH, and dried under high vacuum to give AMC-N3 as a light white solid in 99% yield (286 mg, 787 µmol).

¹H NMR (400 MHz, DMSO-d₆) δ 7.91 (t, J = 6.0 Hz, 1H), 7.83 (d, J = 9.2 Hz, 1H), 7.45 - 7.30 (m, 2H), 7.30 - 7.22 (m, 1H), 7.22 - 7.11 (m, 1H), 6.66 (dd, J = 9.1, 2.6 Hz, 1H), 6.39 (d, J = 2.6 Hz, 1H), 4.68 (s, 1H), 4.36 (d, J = 5.7 Hz, 2H), 3.41 (q, J = 6.9 Hz, 4H), 1.11 (t, J = 6.9 Hz, 6H).

¹³C NMR (101 MHz, DMSO-d₆) δ 162.17, 155.40, 153.93, 150.18, 137.12, 128.73, 128.53, 128.12, 125.04, 123.43, 118.73, 107.79, 102.09, 96.92, 78.72, 43.84, 41.12, 12.33.

### AMC-NH₂

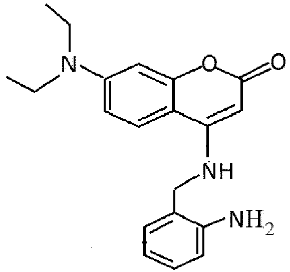

AMC-Cl (50 mg, 0.2 mmol, 1.0 equiv) and 2-(aminomethyl)aniline (97 mg, 0.8 mmol, 4.0 equiv) were suspended in EtOH (2ml) in an 8ml vial and NEt₃ (111 µL, 0.8 mmol, 4.0 equiv) was added. The mixture was heated to 100 °C for 3h and monitored by TLC (EtOAc:hex 50:50). After completion, the reaction mixture diluted with EtOAc (100 ml) and was washed with 0.1M HCI (50 ml) and brine (50 ml). The organic layer was separated, dried over Na2SO4 and evaporated under reduced pressure. The crude product was purified by column chromatography (EtOAc:hex 50:50) to give AMC-NH2 as a white solid (31 mg, 0.1 mmol) in 46% yield.

¹H NMR (400 MHz, Chloroform-d) δ 7.19 (q, J = 9.2, 8.3 Hz, 3H), 6.80 (td, J = 7.4, 1.2 Hz, 1H), 6.78 - 6.75 (m, 1H), 6.49 (s, 2H), 5.29 (s, 1H), 5.01 (t, J = 4.7 Hz, 1H), 4.31 (d, J = 4.5 Hz, 2H), 3.95 (s, 2H), 3.38 (q, J = 7.1 Hz, 4H), 1.18 (t, J = 7.1 Hz, 6H).

¹³C NMR (101 MHz, CDCl₃) δ 164.43, 162.91, 155.92, 154.14, 150.78, 145.26, 130.65, 129.92, 121.31, 120.44, 119.11, 116.54, 108.10, 102.30, 98.36, 81.36, 77.16, 45.14, 44.80, 12.57.

### AMC-PPh₃

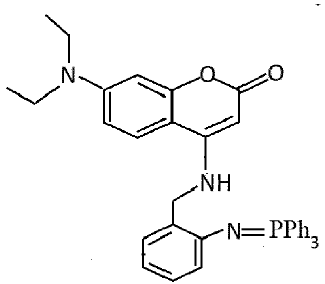

AMC-N₃ (50 mg, 0.14 mmol, 1.0 equiv) and triphenylphosphine (40 mg, 0.15 mmol, 1.1 equiv) were suspended in DMSO (2 mL) in an 8 mL vial. The mixture was heated to 50 °C and monitored by TLC (EtOAc:hex 50:50). After completion, the reaction mixture diluted with EtOAc (50 ml) and was washed with brine (25 mL). The organic layer was separated, dried over Na2SO4 and evaporated under reduced pressure. The crude product was purified by column chromatography (EtOAc:hex 50:50) to give AMC-PPh3 as a off-white solid (72 mg, 0.12 mmol) in 87% yield.

¹H NMR (400 MHz, Chloroform-d) δ 7.74 - 7.66 (m, 6H), 7.56 - 7.49 (m, 3H), 7.46 - 7.39 (m, 6H), 7.20 (ddd, J = 7.4, 2.7, 1.7 Hz, 1H), 6.95 (s, 1H), 6.87 (td, J = 7.6, 1.8 Hz, 1H), 6.71 - 6.62 (m, 2H), 6.47 (dt, J = 8.0, 1.2 Hz, 1H), 6.43 (d, J = 2.6 Hz, 1H), 6.04 (dd, J = 9.0, 2.6 Hz, 1H), 5.31 (d, J = 7.1 Hz, 1H), 4.56 (d, J = 4.5 Hz, 2H), 3.31 (q, J = 7.1 Hz, 4H), 1.13 (t, J = 7.1 Hz, 6H).

¹³C NMR (101 MHz, CDCl₃) δ 164.71, 155.91, 154.14, 150.14, 149.81, 132.49, 132.10, 131.33, 130.54, 130.32, 129.94, 129.03, 128.46, 121.52, 121.27, 117.64, 107.50, 103.37, 98.28, 79.86, 46.88, 44.68, 12.58.

³¹P NMR (162 MHz, CDCl₃) δ 4.68.

### AMC-PPh₂Me

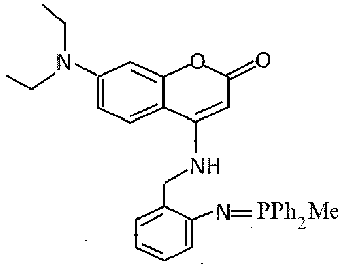

AMC-N₃ (50 mg, 0.14 mmol, 1.0 equiv) and methyldiphenylphosphine (28 µL, 0.15 mmol, 1.1 equiv) were suspended in DMSO (2ml) in an 8 mL vial. The mixture was heated to 50 °C and monitored by TLC (EtOAc). After completion, the reaction mixture diluted with EtOAc (50 ml) and was washed with brine (25 mL). The organic layer was separated, dried over Na2SO4 and evaporated under reduced pressure. The crude product was purified by column chromatography (EtOAc) to give AMC-PPh2Me as a off-white solid (31 mg, 0.06 mmol) in 43% yield.

¹H NMR (400 MHz, DMSO-d6) δ 8.00 (t, J = 5.9 Hz, 1H), 7.91 (ddt, J = 12.0, 6.8, 1.5 Hz, 4H), 7.76 (d, J = 9.1 Hz, 1H), 7.63 - 7.47 (m, 6H), 7.05 (dt, J = 7.6, 2.2 Hz, 1H), 6.77 (td, J = 7.6, 1.8 Hz, 1H), 6.61 (dd, J = 9.1, 2.6 Hz, 1H), 6.49 (td, J = 7.3, 1.1 Hz, 1H), 6.38 (d, J = 2.5 Hz, 1H), 6.32 (dt, J = 7.9, 1.2 Hz, 1H), 5.00 (s, 1H), 4.62 (d, J = 5.8 Hz, 2H), 3.40 (q, J = 7.0 Hz, 4H), 2.32 (d, J = 12.7 Hz, 3H), 1.11 (t, J = 7.0 Hz, 6H).

¹³C NMR (101 MHz, DMSO-d₆) δ 162.44, 155.42, 154.07, 149.99, 149.47, 132.90, 131.90, 131.63, 130.99, 130.89, 130.44, 130.22, 128.88, 128.76, 127.14, 127.05, 123.20, 119.84, 119.72, 116.13, 107.64, 102.46, 96.93, 78.60, 59.75, 43.82, 42.70, 39.52, 20.76, 14.08, 13.18, 12.56, 12.35.

³¹P NMR (162 MHz, DMSO-d₆) δ 3.93.

### AMC-PPhMe₂

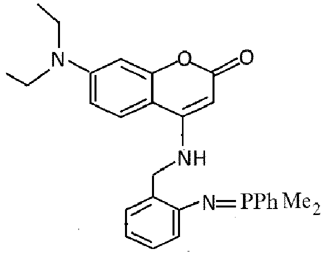

AMC-N₃ (7.8 mg, 1.000 Eq, 21 µmol) and dimethylphenylphosphine (4.3 mg, 4.0 µL, 1.00 Eq, 21 µmol) were suspended in DMSO (1 ml) in an 8ml vial and shaken at rt to give a homogenous solution. The resulting product, AMC-PPhMe2, was used without further purification as a stock solution.

¹H NMR (400 MHz, DMSO-d₆) δ 8.01 (t, J = 6.0 Hz, 1H), 7.88 (ddt, J = 11.6, 6.7, 1.5 Hz, 2H), 7.75 (d, J = 9.1 Hz, 1H), 7.62 - 7.50 (m, 3H), 7.03 (dt, J = 7.7, 2.1 Hz, 1H), 6.81 (td, J = 7.6, 1.8 Hz, 1H), 6.64 (dd, J = 9.1, 2.6 Hz, 1H), 6.48 (td, J = 7.3, 1.1 Hz, 1H), 6.38 (tt, J = 3.2, 1.3 Hz, 2H), 5.03 (s, 1H), 4.48 (d, J = 5.9 Hz, 2H), 3.40 (q, J = 7.0 Hz, 4H), 1.94 (d, J = 13.0 Hz, 6H), 1.11 (t, J = 6.9 Hz, 6H).

¹³C NMR (101 MHz, DMSO-d₆) δ 162.48, 155.41, 153.96, 150.17, 149.98, 133.53, 132.65, 131.45, 131.42, 130.41, 130.31, 130.15, 129.98, 129.75, 128.79, 128.68, 128.31, 128.26, 127.78, 127.31, 127.08, 123.13, 119.54, 119.41, 115.71, 107.66, 102.50, 96.96, 78.63, 43.81, 42.64, 39.52, 15.54, 14.84, 13.95, 13.82, 12.34.

³¹P NMR (162 MHz, DMSO-d₆) δ 8.49.

### AMC-Dye

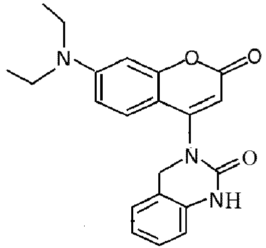

A 25 mL RBF was charged with 4-((2-azidobenzyl)amino)-7-(diethylamino)-2H-chromen-2-one (49.6 mg, 1 equiv, 136 µmol) and was introduced into the glovebox. DMSO (15.0 mL) was added. To the resulting white suspension was added dimethyl(phenyl)phosphane (23.3 µL, 1.2 Eq, 164 µmol) and the resulting mixture was stirred at 25 °C for 30 min to afford a light-yellow solution. A balloon with carbon dioxide was connected and the gas was bubbled through the stirring solution for 2.5 hour (bubbling for 15 min, then kept under CO2 atmosphere). The reaction mixture was diluted with DCM (200 mL), washed with brine 2x 100 mL, dried with Na2SO4, and concentrated. The resulting crude was purified by column chromatography eluting with hexane/EtOAc (1:1 to 0:1). The product was isolated as an off-white solid in 10% yield (5.0 mg, 14 µmol).

¹H NMR (400 MHz, Chloroform-d) δ 7.86 (s, 1H), 7.26 - 7.20 (m, 1H), 7.13-7.09 (m, 1H), 7.04 (td, J = 7.5, 1.1 Hz, 1H), 6.83 - 6.78 (m, 1H), 6.55 - 6.49 (m, 2H), 6.08 (s, 1H), 4.96 (s, 1H), 4.60 (s, 1H), 3.39 (q, J = 7.1 Hz, 4H), 1.18 (t, J = 7.1 Hz, 6H).

¹³C NMR (126 MHz, CDCl3) δ 162.97, 156.68, 154.78, 153.37, 151.17, 136.29, 128.97, 125.99, 125.75, 123.11, 118.26, 114.43, 108.76, 105.65, 104.75, 97.70, 51.39, 44.95, 12.58.

### BODIPY based compounds

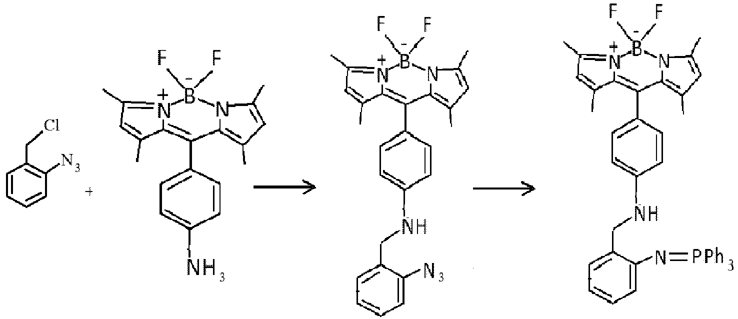

### BODIPY-N₃

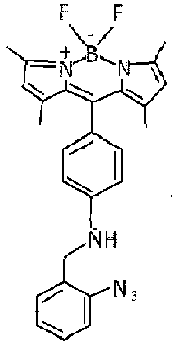

To a 16 mL vial under air were added 1-azido-2-(lodomethyl)benzene (91 mg, 1.2 Eq, 0.35 mmol), 4-(5,5-difluoro-1,3,7,9-tetramethyl-5H-4l4,5l4-dipyrrolo[1,2-c:2',1'-f][1,3,2]diazaborinin-10-yl)aniline (100 mg, 1 Eq, 0.3 mmol), K₂CO₃ (82 mg, 2 Eq, 0.6 mmol) and a magnetic stirring bar. The reaction vessel was cycled three times and THF (6.0 mL, Acros (dry)) were added. The orange-red reaction was stirred at 70 °C for 20 hour. The resulting mixture was filtered over a pad of celite, washed with EtOAc and the filtrate was concentrated. The resulting crude was purified by column chromatography (EtOAc:hex 25:75) to give BODIPY-N3 as an orange solid (97 mg, 0.2 mmol) in 59% yield.

¹H NMR (400 MHz, Chloroform-d) δ 7.34 (td, J = 7.5, 1.3 Hz, 2H), 7.20 (dt, J = 7.3, 1.2 Hz, 1H), 7.14 - 7.07 (m, 1H), 7.04 - 6.98 (m, 2H), 6.74 - 6.68 (m, 2H), 5.96 (d, J = 1.1 Hz, 2H), 4.36 - 4.31 (m, 2H), 4.25 (s, 1H), 2.54 (t, J = 1.1 Hz, 6H), 1.47 (s, 6H).

¹³C NMR (101 MHz, CDCl₃) δ 155.01, 148.58, 143.35, 142.97, 138.23, 132.25, 129.93, 129.60, 129.04, 128.90, 124.96, 123.94, 121.03, 118.40, 113.70, 44.14, 14.80, 14.70.

¹⁹F NMR (376 MHz, Chloroform-d) δ -146.30 (dd, J = 66.6, 33.2 Hz).

¹¹B NMR (128 MHz, Chloroform-d) δ 0.79 (t, J = 33.3 Hz).

### BODIPY-PPh₃

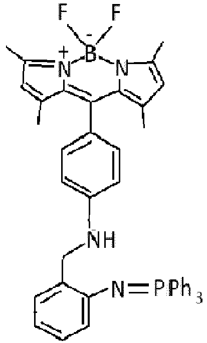

BODIPY-N₃ (30 mg, 0.064 mmol, 1.0 equiv) and triphenylphosphine (17 mg, 0.064 mmol, 1 equiv) were suspended in THF (1 mL) in an 8 mL vial. The mixture was heated to 35°C for 16 hours and monitored by TLC (EtOAc:hex 30:70). After completion, the reaction mixture diluted with DCM (50 ml) and was washed with brine (25 mL). The organic layer was separated, dried over Na2SO4 and evaporated under reduced pressure. The crude product was purified by column chromatography (EtOAc:hex 30:70) to give AMC-PPh3 as an orange solid (42 mg, 0.06 mmol) in 93% yield.

¹H NMR (400 MHz, Chloroform-d) δ 7.80 - 7.70 (m, 6H), 7.57 - 7.49 (m, 3H), 7.43 (tdd, J = 8.2, 2.9, 1.4 Hz, 6H), 7.20 (ddd, J = 7.4, 2.7, 1.8 Hz, 1H), 6.99-6.93 (m, 2H), 6.84 (td, J = 7.6, 1.8 Hz, 1H), 6.73 - 6.66 (m, 2H), 6.63 (td, J = 7.4, 1.2 Hz, 1H), 6.49 (dt, J = 7.9, 1.3 Hz, 1H), 5.96 (s, 2H), 5.34 (s, 1H), 4.53 (s, 2H), 2.55 (s, 6H), 1.50 (s, 6H).

¹³C NMR (101 MHz, CDCl3) δ 154.78, 149.95, 149.62, 143.62, 143.44, 132.65, 132.55, 132.36, 132.19, 131.98, 131.95, 131.77, 130.78, 129.51, 129.49, 128.93, 128.81, 128.75, 127.75, 122.93, 121.41, 121.31, 120.92, 117.47, 114.10, 47.58, 14.89, 14.70.

³¹P NMR (162 MHz, Chloroform-d) δ 2.73.

¹⁹F NMR (377 MHz, Chloroform-d) δ -146.30 (dd, J = 66.7, 32.8 Hz).

¹¹B NMR (128 MHz, Chloroform-d) δ 0.82 (t, J = 33.4 Hz).

### BODIPY-DYE

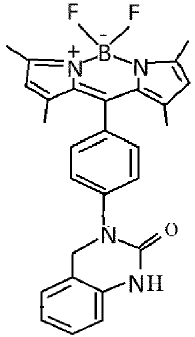

An 18 mL vial a magnetic stirring bar was charged with N-(2-azidobenzyl)-4-(5,5-difluoro-1,3,7,9-tetramethyl-5H-4l4,5l4-dipyrrolo[1,2-c:2',1'-f][1,3,2]diazaborinin-10-yl)aniline (30.2 mg, 1 Eq, 64.2 µmol) and was introduced into the Glovebox. Then, DMSO (1.0 mL, Acros) was added. To the resulting partially dissolved red solution was added dimethyl(phenyl)phosphane (10.6 mg, 11 µL, 1.2 Eq, 77.1µmol) and the resulting mixture was stirred at 25 °C for 2 min. Then a balloon with carbon dioxide (2.83 mg, 1 Eq, 64.2 µmol) was connected and the gas was bubbled through the stirring solution for 2.5 hour (bubbling for 15 min, then under CO2 atmosphere). The dark red solution brightens to bright orange within minutes. Diluted with DCM (100 mL), washed with brine 2x 20 mL, dried Na2SO4. The resulting crude was purified by column chromatography eluting with hexane/EtOAc (3:1 to 1:1 to 0:1) to give an orange solid in 70% yield (21 mg, 45 µmol).

¹H NMR (500 MHz, DMSO-d₆) δ 9.73 (s, 1H), 7.61 - 7.55 (m, 2H), 7.40 - 7.33 (m, 2H), 7.27 - 7.19 (m, 2H), 6.94 (td, J = 7.4, 1.2 Hz, 1H), 6.92 - 6.89 (m, 1H), 6.22 - 6.19 (m, 2H), 4.91 (s, 2H), 2.46 (s, 6H), 1.43 (s, 6H).

¹³C NMR (126 MHz, DMSO-d₆) δ 154.81, 152.95, 143.17, 142.78, 141.85, 137.09, 130.86, 129.95, 128.72, 128.09, 127.76, 125.67, 124.69, 124.40, 122.29, 121.46, 121.37, 118.60, 113.58, 113.41, 67.48, 49.70, 14.21.

¹⁹F NMR (471 MHz, DMSO-d₆) δ -143.62 (dd, J = 65.9, 31.8 Hz).

¹¹B NMR (160 MHz, DMSO-d₆) δ 0.60 (t, J = 33.0 Hz).

### Pyrone based compounds

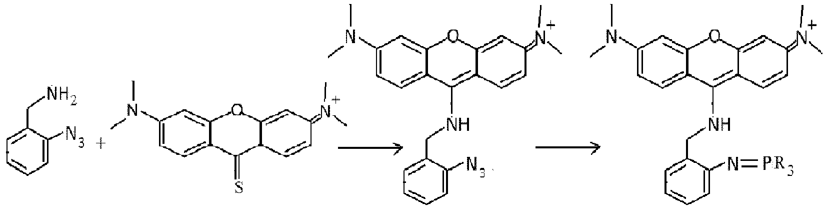

### PYR-N₃

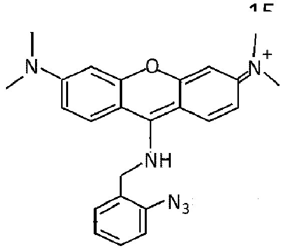

3,6-bis(dimethylamino)-9H-xanthene-9-thione (130.0 mg, 1 equiv, 0.43 mmol) was dissolved in DCM (20 mL) and was cooled to 0 °C, after which triflic anhydride (160 µL, 2.2 Eq, 0.95 mmol) was added dropwise at 0 °C via syringe and the resulting deep blue mixture was stirred at 0 °C for 10min. Then, (2-Azidophenyl)methanamine2,3 (266 mg, 4 Eq, 1.7 mmol) was added at 0 °C and the resulting mixture was stirred at room temperature overnight. The reaction mixture was diluted with DCM (150 ml) and was washed with 1M HCl (50 ml) and brine (50 ml). The organic layer was separated, dried over Na2SO4 and evaporated under reduced pressure. The crude product was purified by purified by preparative RP-HPLC (gradient of 30%-100% of MeCN in water [0.1% TFA]) to give PYR-N3 as an orange solid (140 mg, 0.28 mmol) in 65% yield.

¹H NMR (400 MHz, Methanol-d4) δ 7.99 (s, 2H), 7.52 - 7.47 (m, 1H), 7.44 (dd, J = 7.9, 1.4 Hz, 1H), 7.40 (dd, J = 8.0, 1.2 Hz, 1H), 7.23 (td, J = 7.5, 1.2 Hz, 1H), 6.94 (d, J = 9.7 Hz, 2H), 6.75 (d, J = 2.6 Hz, 2H), 5.13 (s, 2H), 3.20 (s, 12H).

¹³C NMR (101 MHz, MeOD) δ 157.20, 157.06, 139.25, 131.01, 129.27, 128.19, 128.14, 126.50, 119.85, 97.91, 40.22.

### PYR-PPh₃

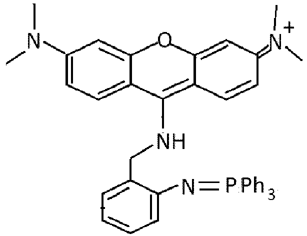

**PYR-N₃** (20 mg, 0.039 mmol, 1.0 equiv) and triphenylphosphine (12 mg, 0.045 mmol, 1.15 equiv) were suspended in DMSO (1 mL) in a 4 mL vial. The mixture was heated to 50 °C and monitored by RP-HPLC (EtOAc:hex 50:50). After completion, the reaction was loaded on a short silica ped and washed with EtOAc (50 mL) to remove unreacted phosphine. Next, the pad was washed with MeOH to elute the crude product. Then, the solvent was evaporated, and the crude product was purified by preparative RP-HPLC (gradient of 30%-100% of MeCN in water [0.1% TFA]) to give PYR-PPh3 as an orange solid (12 mg, 0.016 mmol) in 41% yield.

¹H NMR (500 MHz, Methanol-d4) δ 7.91 (dddd, J = 8.7, 5.9, 3.7, 1.4 Hz, 4H), 7.77 - 7.68 (m, 12H), 7.50 (dd, J = 7.7, 1.6 Hz, 1H), 7.33 (tt, J = 7.5, 1.1 Hz, 1H), 7.26 (td, J = 7.7, 1.7 Hz, 1H), 7.14 (dt, J = 7.8, 1.3 Hz, 1H), 6.84 (s, 2H), 6.77 (d, J = 2.6 Hz, 2H), 5.21 (s, 2H), 3.20 (s, 12H).

¹³C NMR (126 MHz, MeOD) δ 157.17, 157.09, 136.93, 136.91, 135.02, 134.94, 134.34, 134.29, 131.51, 131.40, 130.70, 130.40, 128.97, 128.26, 124.18, 122.23, 121.41, 98.00, 49.00, 40.28.

³¹P NMR (162 MHz, Methanol-d4) δ 35.93.

¹⁹F NMR (471 MHz, Methanol-d4) δ -77.01.

### PYR-NH₂

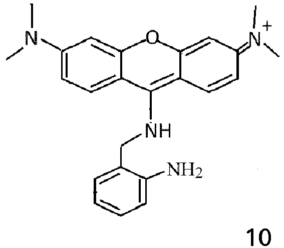

3,6-bis(dimethylamino)-9H-xanthene-9-thione (50 mg, 1 equiv, 0.17 mmol) was dissolved in DCM (20 mL) and was cooled to 0 °C, after which triflic anhydride (62 µL, 2.2 Eq, 0.37 mmol) was added dropwise at 0 °C via syringe and the resulting deep blue mixture was stirred at 0 °C for 10 min. Then, (2-Azidophenyl)methanamine2,3 (82 mg, 4 equiv, 0.66 mmol) was added at 0 °C and the resulting mixture was stirred at room temperature overnight. The reaction mixture was diluted with DCM (150 ml) and was washed with 1M HCI (50 ml) and brine (50 ml). The organic layer was separated, dried over Na2SO4 and evaporated under reduced pressure. The crude product was purified by preparative RP-HPLC (gradient of 30%-100% of MeCN in water [0.1% TFA]) to give PYR-NH2 as an orange solid (12 mg, 0.28 mmol) in 15% yield.

¹H NMR (400 MHz, Methanol-d4) δ 7.78 (dd, J = 7.7, 1.6 Hz, 1H), 7.71 (d, J = 9.6 Hz, 2H), 7.66 (td, J = 7.6, 1.3 Hz, 1H), 7.59 (td, J = 7.7, 1.6 Hz, 1H), 7.44 (dd, J = 7.9, 1.4 Hz, 1H), 6.85 (dd, J = 9.6, 2.7 Hz, 2H), 6.78 (d, J = 2.6 Hz, 2H), 4.33 (s, 2H), 3.21 (s, 12H).

¹³C NMR (126 MHz, MeOD) δ 158.90, 157.32, 155.66, 140.59, 132.31, 131.76, 131.12, 130.71, 129.30, 128.67, 112.74, 103.89, 97.70, 40.31.

### PYR-DYE

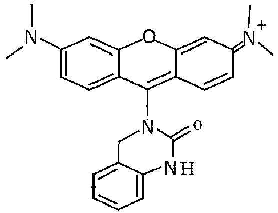

An 18 mL vial a magnetic stirring bar was charged with **PYR-N₃** (15.0 mg, 1 Eq, 0.029 mmol). Then, DMSO (4.0 mL) was added and the resulting partially dissolved orange solution was added dimethyl(phenyl)phosphane (4.72 mg, 4.86 µL, 1.2 Eq, 34.2 µmol). The resulting mixture was stirred at room temperature for 5 min. Then a balloon with carbon dioxide (1.25 mg, 1 Eq, 28.5 µmol) was connected and the gas was bubbled through the stirring solution for 2.5 hour (bubbling for 15 min 15:18-33, then kept under CO2 atmosphere). The orange solution becomes magenta within minutes. The crude reaction was diluted with water (10 mL) and the mixture was subjected to separation using preparative RP-HPLC (gradient of 30%-100% of MeCN in water [0.1% TFA]) to give PYR-Dye as a magenta solid (8 mg, 0.016 mmol) in 54% yield.

¹H NMR (400 MHz, Methanol-d4) δ 7.82 (d, J = 9.5 Hz, 2H), 7.37 - 7.30 (m, 1H), 7.25 - 7.17 (m, 3H), 7.09 (td, J = 7.5, 1.2 Hz, 1H), 7.06 - 7.02 (m, 1H), 6.99 (d, J = 2.4 Hz, 2H), 4.98 (s, 2H), 3.35 (s, 12H).

¹³C NMR (101 MHz, MeOD) δ 160.90, 159.28, 155.45, 154.86, 138.16, 130.05, 129.54, 126.81, 124.10, 118.77, 116.22, 115.73, 113.66, 97.88, 53.86, 49.00, 41.07.

¹⁹F NMR (377 MHz, Methanol-d4) δ -77.20.

## Claims

1. Process for the detection of CO₂ in a sample comprising the steps of
- supplying a reagent comprising a reactive moiety toward carbon dioxide and a dye moiety Dy, wherein the dye moiety Dy has first optical and/or spectral properties;
- contacting said sample with said reagent so that the reagent reacts with carbon dioxide to lead to a reacted agent, wherein the dye moiety Dy has second optical and/or spectral properties different from the first optical and/or spectral properties; and
- detecting one or both of the first and the second optical and/or spectral properties of the dye moiety Dy.

2. Process according to claim 1, wherein said reagent is a compound of Formula (I') 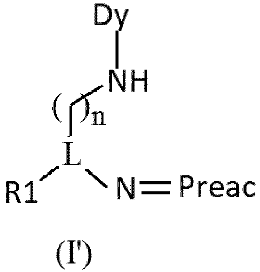 and the reacted reagent is a compound of Formula (II'): Wherein : 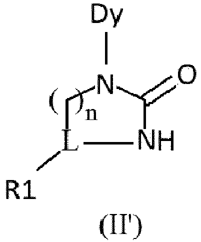
- Dy denotes a dye moiety having first optical properties in the compound of Formula (I') and second optical properties in the compound of Formula (II'),
- Preact denotes a phosphorous based reactive moiety, on which CO₂ reacts in an irreversible manner,
- R1 denotes from 1 to 4 groups independently selected from, H, C₁-C₆ alkyl, C₁-C₆ alkenyl, C₁-C₆ alkynyl, or denotes 2 groups forming a fused saturated, non-saturated or aromatic C₄-C₈ ring with the spacer L to which they are linked, wherein from 1 to 3 carbon atom, where applicable, are independently from each other optionally substituted by a group selected from -COO-, -CO-, NH-, -O-, -S- or substituted with a group selected from -OH, SH, NH₂, NO₂, halogens, or denotes a macromolecule such as a DNA, RNA, peptide, protein, enzyme,
- L denotes an internal spacer selected from a linear or branched carbon chain comprising from 1 to 10, preferably from 1 to 6 carbon atoms being either saturated or mono- di- or tri-unsaturated, or a cyclic chain comprising from 4 to 7, or from 5 to 6 carbon atoms, being either saturated or mono- or di- unsaturated, or an aromatic group such as a phenyl, or a heteroaromatic group such as pyridine, (benzo)furan, (benzo)thiophene, or pyrrole, wherein, where applicable, in the flexible linear or branched carbon chain and in the non-aromatic cyclic chain, from 1 to 3 carbon atoms, can independently from each other optionally be replaced by a heteroatom selected from -O-, -S-, -NH-,-CO-, -NHCO- or -COO- or substituted by a group -OH, -NH₂, halogen, NO₂, or alkyl comprising from 1 to 3 carbon atoms, and
- n denotes an integer selected from 0, 1, 2 or 3.

3. Process according to claim 1 wherein the dye moiety Dy comprises at least one double bond in position α to the nitrogen atom to which it is bounded, so that said double bond is conjugated with the free lone pair of the nitrogen atom in Formula (I').

4. Process according to one of claim 1 and 2, wherein the dye moiety Dy is selected from one of the following moieties Dy1, Dy2 and Dy3:

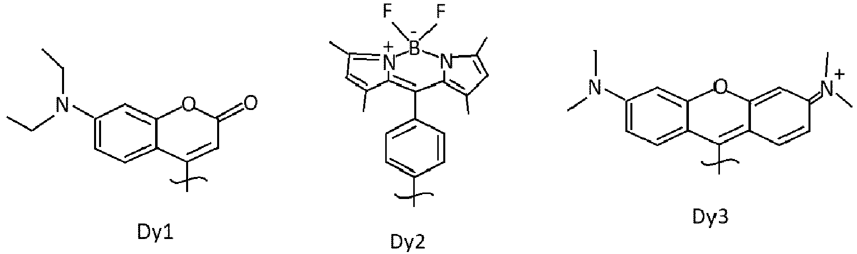

5. Process according to one of claims 1 to 3 wherein the group Preact denotes a phosphine selected from PMePh₂, PMe₂Ph and PPh₃ wherein "Ph" independently denotes an unsubstituted phenyl group or a phenyl group substituted with 1 to 4 substituents selected from C₁-C₆ alkyl, C₂-C₆ alkenyl, C₂-C₆ alkynyl, NO₂, halogen and CF₃.

6. Process according to one of claims 1 to 4, wherein the reaction between CO₂ and a compound of formula (I) provides an intermediate compound of Formula (III'), which immediately provides an intramolecular reaction leading to a compound of Formula (II'). 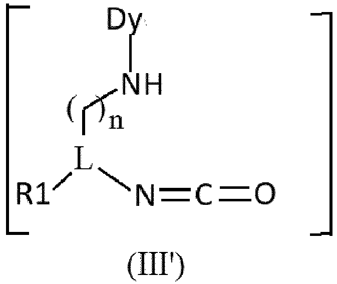 Wherein Dy, L, R1 and n are as above defined.

7. Process according to one of claims 1 to 6, wherein said first and second optical properties comprises one or more of a fluorescence emission, a light emission, a light absorption at a specific wavelength, said wavelength being different for the first optical properties and for the second optical properties.

8. Process according to one of claims 1 to 7, wherein at least one of said first and second optical properties is correlated to the amount of CO₂ in the tested sample.

9. Process according to one of claims 1 to 8, wherein the sample is selected from a liquid sample, an air sample or a gas sample.

10. Process according to one of claims 1 to 9, wherein the sample is a biological sample, a biochemical sample, a chemical sample, and wherein said process allows to sense the variation of carbon dioxide over time.

11. A compound of Formula (I') 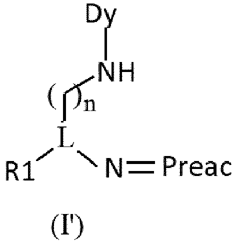 Wherein :
- Dy denotes a dye moiety having first optical properties in the compound of Formula (I) and second optical properties in the compound of Formula (II),
- Preact denotes a phosphorous based reactive moiety, on which CO₂ reacts in an irreversible manner,
- R1 denotes from 1 to 4 groups independently selected from, H, C₁-C₆ alkyl, C₁-C₆ alkenyl, C₁-C₆ alkynyl, or denotes 2 groups forming a fused saturated, none saturated or aromatic C₄-C₈ ring with the phenyl group to which they are linked, and
- L denotes an internal spacer selected from a linear, branched or cyclic carbon chain comprising from 1 to 10, preferably from 1 to 6 carbon atoms being either saturated or mono- di- or tri-unsaturated, or an aromatic group selected from a phenyl, or a pyridine group, and
- n denotes an integer selected from 0, 1, 2 or 3.

12. A compound according to claim 11, wherein the compound is selected from the followings:

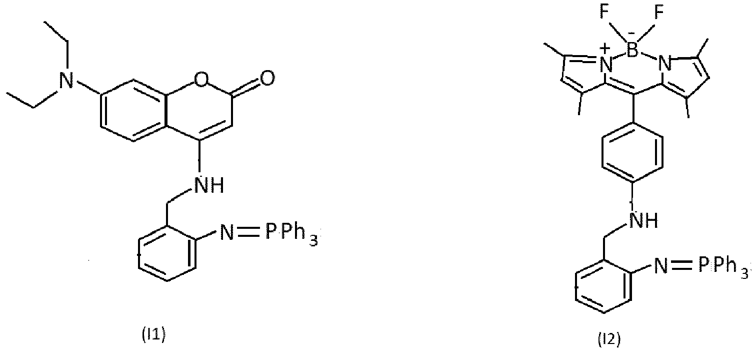

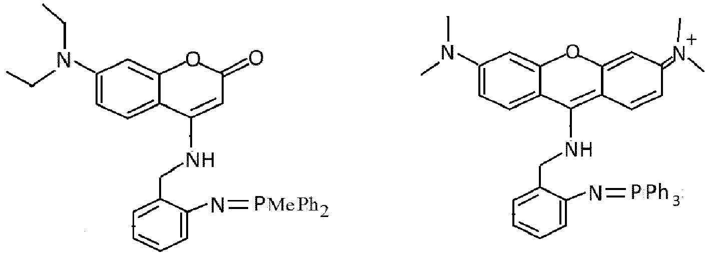

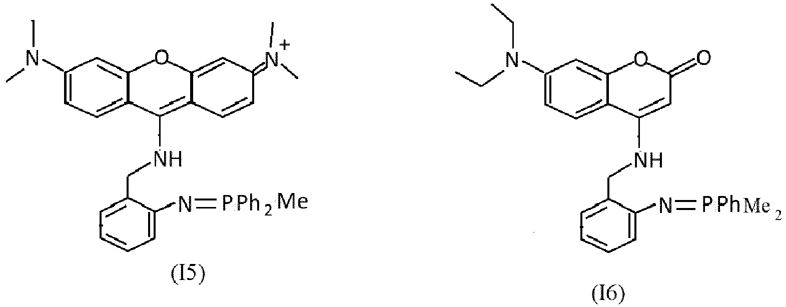

13. A process to produce a compound of Formula (I') according to one of claims 11 and 12, the process comprising the step of reacting a compound of Formula (la') : 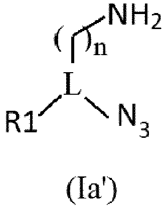 With a compound of Formula (lb) : 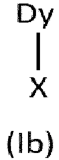 to provide a compound of Formula (Ic'): 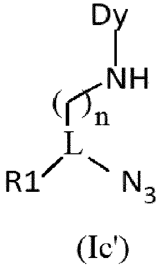 Wherein Dy, R1, L and n are as defined above and wherein X denotes a suitable electrophile such as halogens, a carbonyl, a thiocarbonyl, a triflate, a mesylate and related electrophiles,
in the presence of triethylamine in ethanol at a temperature comprised between 60°C and 120°C during 10 minutes to 20 hours.

14. Process according to claim 13, further comprising the step of reacting a compound of Formula (Ic') according to claim 12 with a phosphorous based moiety Preac, in DMSO at a temperature comprised between 20°C and 80°C to obtain a compound of Formula (I') above wherein Dy, R1, L and Preac are as defined therein.

15. A kit for testing the presence and/or the amount of carbon dioxide in a sample, comprising at least one compound of Formula (I') according to claims 10 to 13 or subparts thereof separately conditioned.
